# DEMANDE DE BREVET EUROPEEN

(11) **EP 0 987 013 A1**
(43) Date de publication de la demande: **22.03.2000**
(21) Numéro de dépôt: 99402059.2
(22) Date de dépôt: 13.08.1999
(51) Int. Cl.: A61K 7/48, A61K 7/02

(54) **Composition topique comprenant un agent gélifiant de type polyester sulfoné et un agent de structuration cationique**

(30) Priorité: 14.09.1998 FR 9811434
(71) Demandeur: L'OREAL, 75008 Paris (FR)
(72) Inventeur: Ferrari, Véronique, 94700 Maisons Alfort (FR)
(74) Mandataire: Lhoste, Catherine

(57) **Abrégé**

L'invention se rapporte à une composition à application topique comprenant une phase aqueuse, un agent gélifiant hydrophile de type polyester sulfoné et un agent de structuration cationique choisi parmi les cations monovalents, multivalents, les polycations et leurs associations, ce polyester étant un oligomère copolyester téréphtalique hydrosoluble ou hydrodispersable de masse moléculaire, en poids, inférieure à 20 000 et comprenant essentiellement des motifs répétitifs dicarboxylates de formule (I):

[-CO-A-CO-O-(CH₂-CH₂O)ₙ-] (I)

dans laquelle
- A représente un groupement 1,4-phénylène, sulfo-1,3-phénylène et éventuellement 1,3-phénylène,
- n va de 1 à 4,
- au moins 35 % en mole desdits motifs de formule (I) étant des motifs pour lesquels A représente un groupement 1,4-phénylène et n est égal à 1,
- au moins 7 % en mole desdits motifs de formule (I) étant des motifs pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène.

Cette composition peut être un rouge à lèvres, un fond de teint, un produit anti-cernes, un produit déodorant ou de protection solaire, à propriétés sans transfert.

## Description

La présente invention se rapporte à une composition aqueuse à application topique comprenant un gélifiant de phase aqueuse particulier, destinée en particulier aux domaines cosmétique et/ou dermatologique. Elle peut se présenter sous forme de pâte souple ou coulé en stick ou en coupelle et constituer un produit de soin, de traitement et/ou de maquillage de la peau, y compris du cuir chevelu, des lèvres et/ou des phanères comme les cils, les ongles, les cheveux. Cette composition possède une bonne stabilité dans le temps, notamment à la chaleur, et permet l'obtention d'un film présentant des propriétés de sans transfert et un aspect brillant, associés à une sensation de fraîcheur et de confort.

Cette composition peut constituer un fond de teint, un rouge à lèvres, un eye liner, un mascara, un produit anti-cernes, de protection solaire, de bronzage artificiel de la peau ou de maquillage du corps mais aussi un fard à paupières ou à joues, un déodorant.

Les produits de maquillage ou de soin de la peau ou des lèvres des êtres humains comme les fonds de teint ou les rouges à lèvres contiennent généralement des phases grasses telles que des cires et des huiles, des pigments et/ou charges et, éventuellement des additifs comme des actifs cosmétiques ou dermatologiques.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

Pour remédier à ces inconvénients, les cosméticiens ont envisagés l'association d'huiles volatiles et notamment de silicones volatiles ou d'isoparaffines avec des résines siloxysilicate à structure tridimensionnelle. De telles associations sont notamment décrites dans les documents JP-A-61-65809 et EP-A-602905.

Ces compositions, bien que présentant des propriétés de "sans transfert" améliorées ont l'inconvénient de laisser sur les lèvres ou la peau, après évaporation des huiles de silicone, un film qui devient inconfortable au cours du temps (sensation de dessèchement et de tiraillement), écartant un certain nombre de femmes de ce type de rouge à lèvres ou de fonds de teint. En outre, ces compositions sont très glissantes, rendant leur application souvent mal aisée et elles laissent sur les lèvres un film mat. Or, les consommatrices sont toujours à la recherche d'un produit sans transfert brillant. De plus, ces films sont souvent difficiles à démaquiller, nécessitant souvent un produit démaquillant spécial.

L'invention a justement pour objet une composition à application topique contenant un gélifiant aqueux et un agent de structuration particuliers permettant notamment de remédier à ces inconvénients.

De façon plus précise, l'invention à pour objet une composition à application topique comprenant une phase aqueuse, un agent gélifiant hydrophile de type polyester sulfoné et un agent de structuration cationique choisi parmi les cations monovalents, multivalents, les polycations et leurs associations, ce polyester étant un oligomère copolyester téréphtalique hydrosoluble ou hydrodispersable de masse moléculaire, en poids, inférieure à 20 000 et comprenant essentiellement des motifs répétitifs dicarboxylates de formule (I):

[-CO-A-CO-O-(CH₂-CH₂O)ₙ-] (I)

dans laquelle
- A représente un groupement 1,4-phénylène, sulfo-1,3-phénylène et éventuellement 1,3-phénylène,
- n va de 1 à 4,
- au moins 35 % en mole desdits motifs de formule (I) étant des motifs pour lesquels A représente un groupement 1,4-phénylène et n est égal à 1,
- au moins 7 % en mole desdits motifs de formule (I) étant des motifs pour lesquels A représente un groupement sulfo-1,3-phénylène,
- et éventuellement jusqu'à 20 % en mole, de préférence jusqu'à 0,5 % à 5 % en mole, de motifs de formule (I) pour lesquels A représente un groupement 1,3-phénylène.

Ce gélifiant n'est pas un pigment dispersible dans l'eau. Il ne sert pas non plus à enrober ou traiter des pigments mais à structurer en particulier sous forme de stick une composition aqueuse.

Cette composition peut être une composition cosmétique, dermatologique, pharmaceutique ou hygiénique présentant en particulier l'avantage de conduire à un dépôt brillant, de très bonne tenue, ne transférant pas du tout, résistant à l'eau, tout en étant très agréable à l'application et à porter tout au long de la journée. Le dépôt est notamment ni gras, ni sec, et non collant. En outre la composition de l'invention présente des propriétés de confort à l'application et au cours du temps et notamment des propriétés de fraîcheur à l'application, d'hydratation et d'absence de sensations de tiraillement ; elle est facile à démaquiller avec les démaquillants habituellement utilisés. De plus, la composition est moins glissante que les produits sans transfert contenant des huiles volatiles, facilitant ainsi son application sur les lèvres et la peau. Elle est, en outre stable dans le temps et en particulier à I température, ce qui permet son utilisation dans les pays chauds.

De manière préférentielle, au moins 40 % en mole, et mieux de 40 à 90 % en mole des motifs de formule (I) sont des motifs pour lesquels A représente un groupement 1,4-phénylène et n est égal à 1.

De préférence, au moins 10 % en mole, et plus préférentiellement de 10 % à 25 % en mole des motifs de formule (I) sont des motifs pour lesquels A représente un groupement sulfo-1,3-phényléne.

Les extrémités des chaînes dudit oligomère copolyester peuvent être semblables ou différentes et représentées essentiellement par les groupements de formule (I'):

-CO-A-CO-O-(CH₂-CH₂-O)ₙ-H (I')

dans laquelle A et n sont définis ci-dessus.

L'oligomère peut également présenter en extrémités de chaîne, et en quantité mineure, des groupements de formules ; -A-CO-OH ou -A-CO-OR : formules dans lesquelles A est défini comme ci-dessus et R représente un groupement alkyle en C₁-C₄.

Lorsque A représente un groupement sulfo-1,3-phénylène, il s'agit plus particulièrement d'un sulfonate de métaux alcalins, notamment de sodium ou de potassium, ou d'un sulfonate d'ammonium ou de mono-, di-, tri- ou tétra- alkylammonium inférieur. Par alkylammonium inférieur, on entend de préférence un ammonium dont le ou les radicaux alkyles sont des alkyles inférieurs, de préférence en C₁-C₆. De manière préférentielle, il s'agit d'un sulfonate de sodium.

L'oligomère copolyester peut comprendre éventuellement jusqu'à 20 % en mole, de préférence de 0,5 à 5 % en mole de motifs de formule (I) pour lesquels A représente un groupement 1,3-phénylène.

De préférence, l'oligomère copolyester de l'invention a une masse moléculaire, en poids, allant de 5 000 et 14 000, et plus préférentiellement de 8 000 à 10 000. Les masses moléculaires en poids sont mesurées par chromatographie par perméation de gel dans le diméthylacétamide contenant 10⁻² N de LiBr, à 100°C. Les résultats sont exprimés en équivalents polystyrène.

L'oligomère préféré est en particulier celui décrit dans l'exemple 1 ci-après.

L'oligomère copolyester peut être obtenu par les procédés usuels de préparation des polyesters, par voie fondue, voie solvant ou voie interfaciale, procédés faisant intervenir des réactions
. d'estérification de diacides et de diols et polycondensation,
. de transestérification de diesters et de diols et polycondensation,
. d'autocondensation d'hydroxyacides,
. de Schotten-Baumann par mise en oeuvre de diols et de chlorures d'acide puis polycondensation,
. de polymérisation de lactones,
en contrôlant la teneur minimum en motifs de formule (I) pour lesquels A représente un groupement 1,4-phénylène et n est égal à 1, semblables par les rapports stoechiométriques initiaux des différents monomères et par la maîtrise des réactions secondaires.

Un mode de préparation particulièrement intéressant est celui par transestérification/ polycondensation et/ou d'estérification/polycondensation par voie fondue à l'aide d'un catalyseur de transestérification et/ou estérification.

La maîtrise de la structure est obtenue par contrôle de la teneur minimum en motifs de formule (I) pour lesquels A représente un groupement 1,4-phénylène et n est égal à 1, semblables par les rapports stoechiométriques initiaux des différents monomères diacides et/ou diesters et diols et par mise en oeuvre d'un agent limiteur d'éthérification, agent limiteur qui peut être un composé basique tel que les amines aliphatiques ou aromatiques ou un hydroxyde ou acétate de métaux alcalins ou alcalino-terreux.

Le contrôle de la masse moléculaire est obtenu d'une manière connue de l'homme du métier, par compromis adéquat entre la pression, la température et le temps.

L'oligomère copolyester téréphtalique utilisé selon l'invention peut être préparé par estérification et/ou transestérification/polycondensation d'une composition monomère à base :
- d'acide, anhydride ou diester téréphtalique (Tp)
- d'acide, anhydride ou diester sulfo-isophtalique (Slp)
- éventuellement d'acide, anhydride ou diester isophtalique (Ip)
- et d'éthylène glycol (EG)
selon des quantités relatives correspondant à
∗ un rapport molaire (Slp)/[(Tp)+(Slp)+(lp)] d'au moins 7/100, de préférence d'au moins 10/100, tout particulièrement de 10/100 à 25/100
∗ un rapport molaire (Ip)/[(Tp)+(Slp)+(lp)] de 20/100 au plus, de préférence de 5/100 au plus
∗ un rapport molaire (EG)/[(Tp)+(Slp)+(Ip)] de 2/1 à 3/1.
en présence d'un catalyseur d'estérification et/ou transestérification et d'un limiteur de d'éthérification.

Le monomère téréphtalique (Tp) est de préférence mis en oeuvre sous la forme de diester inférieur (diester de dialkyle en C₁-C₄), de diméthyle de préférence. Le monomère sulfo-isophtalique (Slp) est de préférence mis en oeuvre sous la forme d'un sulfonate de métal alcalin (sodium notamment) de diester inférieur (d'alkyle en C₁-C₄), de méthyle de préférence. On peut citer tout particulièrement le sodio-oxysulfonyl-5 isophtalate de diméthyle. Le monomère isophtalique (Ip) éventuel est de préférence mis en oeuvre sous la forme d'acide isophtalique.

Lorsque tous les monomères "diacides" sont mis en oeuvre sous la forme d'un diesters, l'opération de transestérification (interéchange) entre ces monomères "diacides" et l'éthylène glycol est effectuée à une température supérieure ou égale à 130°C, de préférence de l'ordre 140 à 220°C et tout particulièrement de l'ordre de 180 à 220°C ; à cette température le méthanol (cas préférentiel des diesters méthyliques) formé est éliminé du milieu réactionnel de préférence par distillation. Cette opération d'interéchange est réalisée en présence d'un catalyseur de transestérification métallique et d'un limiteur d'éthérification. Elle dure notamment de 1 à 4 heures et généralement de 2 à 3 heures.

Le catalyseur est de préférence un carboxylate métallique tel que l'acétate de manganèse, l'acétate de zinc, l'acétate de cobalt ou l'acétate de calcium, ou d'un titanate organique ou minéral tel que le titanate de butyle, le titanate de nitrilo-2,2',2"-triéthyle (ou aminotriéthanolate de titane jouant en outre le rôle de limiteur d'éthérification) ou le titanate de calcium. Les catalyseurs préférés sont les titanates organiques ; ils sont mis en oeuvre en quantité de l'ordre d'au moins 0,001 % en poids exprimé en titane, de préférence de l'ordre de 0,002 % à 0,02 % en poids de titane par rapport au poids de réactifs présents.

L'agent limiteur d'éthérification peut être un composé basique tel que les amines aliphatiques ou aromatiques (triéthanolamine, carbonate de guanidine, diméthylaniline, naphtylamine ...) ou un hydroxyde ou acétate de métaux alcalins ou alcalino-terreux (acétate de sodium, potassium, benzoate de sodium ...). Il est mis en oeuvre généralement en quantité de l'ordre de 0,001 % à 0,05 % par rapport au poids de réactifs présents.

Lorsque plus de 90% de la quantité théorique de méthanol ont été distillés, le polyol excédentaire est éliminé en portant la température du milieu réactionnel à 230°C.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression qui peut aller jusqu'à 10 Pa ; une réduction de pression jusqu'à 10 millibars environ dure de l'ordre de 40 min.

L'opération de polycondensation se déroule avec élimination de molécules de polyol, cette opération est stoppée lorsque le couple moteur de l'arbre d'agitation indique une valeur équivalente à environ 0,5 à 5 mètres.newton pour une température de 250°C de la masse réactionnelle et une vitesse d'agitation de 80 tr/min d'un mobile en forme d'ancre dans un réacteur de 7,5 litres. Le vide est ensuite cassé à l'azote, et le polymère est coulé dans une lingotière ; après refroidissement, le polymère est broyé.

Lorsque l'un des monomères "diacides" est présent sous forme diacide ou anhydride et le ou les autres sous forme de diester(s), lesdits oligomères copolyesters sont obtenus en réalisant d'abord une opération de transestérification des monomères diesters avec de l'éthylène glycol dans les conditions décrites ci-dessus, suivie d'une opération d'estérification dans le milieu du monomère diacide ou anhydride avec de l'éthylène glycol, puis polycondensation dans les conditions décrites ci-dessus, la quantité totale d'éthylène glycol étant répartie entre les deux opérations (transestérification et estérification).

Si nécessaire, l'opération d'estérification est réalisée par ajout dans le milieu réactionnel résultant de l'opération de transestérification, du monomère sous forme diacide ou anhydride et d'éthylène glycol préalablement mis en suspension, à une température correspondant à celle de la fin de la température d'interéchange ; la période d'introduction est de l'ordre de 1 heure. Cette opération d'estérification est réalisée à une température de l'ordre de 230 à 280°C, de préférence de l'ordre de 250 à 260°C, en présence d'un catalyseur du même type que celui de transestérification et d'un agent limiteur d'éthérification.

L'opération est réalisée en présence des mêmes types de catalyseur et de limiteur d'éthérification que ceux mis en oeuvre lors de l'opération de transestérification, et ce dans les mêmes proportions. La réaction s'effectue avec élimination d'eau qui est soutirée du réacteur en même temps que le polyol en excès.

Ce type de procédé de préparation est notamment décrit dans la demande de brevet WO-A-95/32997.

Le gélifiant hydrophile peut être présent dans la composition selon l'invention en une teneur allant de 0,5 % à 40 % en poids, par rapport au poids total de la composition, de préférence de 5 % à 40 % et mieux de 15 à 25 %. Pour une teneur en gélifiant hydrophile au moins égale à 5 %, on obtient une composition solide qui peut être coulée sous forme de stick ou de coupelle.

La composition de l'invention peut comprendre, en outre, selon l'application spécifique envisagée, tout additif couramment utilisé dans les domaines à application topique notamment dermatologique et cosmétique. On peut citer, à titre d'exemple d'additifs, les matières colorantes comme les colorants solubles dans le milieu de la composition, les pigments et les nacres, les agents de protection solaire, les charges, les antioxydants, les conservateurs, les agents épaississants, les agents plastifiants, les tensioactifs, les cires, les huiles, les parfums, les actifs cosmétiques ou dermatologiques comme les agents hydratants. Ces additifs sont utilisés dans des concentrations classiquement utilisées dans les domaines considérés. Bien entendu, l'homme du métier veillera à choisir ce ou ces éventuels additifs et/ou leur quantité, de telle manière que les propriétés avantageuses de la composition selon l'invention ne soient pas, ou substantiellement pas, altérées par l'adjonction envisagée.

Les inventeurs ont trouvé que l'introduction, à l'oligomère défini ci-dessus, de certains additifs tels que des gélifiants classiques, des huiles, des pigments ou des charges, pouvait modifier la structuration de la composition. Ceci est notamment remarquable, lorsque la composition est sous forme solide et notamment sous forme de bâton ou de stick. En particulier, la dureté du stick diminue. Par ailleurs, dans certains cas, les propriétés du film comme la brillance, déposé notamment sur la peau, les lèvres ou les phanères sont modifiées.

De façon surprenante, les inventeurs ont trouvé que les agents de structuration cationiques comme les cations monovalents ou multivalents, les polycations et leurs associations permettaient de conserver les propriétés de rigidité d'origine de la composition de l'invention.

Par "cation multivalent", on entend un cation chargé au moins 2 fois et notamment chargé 2, 3, 4 fois ou plus.

De façon avantageuse, la force ionique de l'agent de structuration cationique, qui est fonction de la charge du ou des cations, de leur taille et de la quantité de cations présents, est élevée. En particulier, la quantité de cations ou polycations libres dans l'agent de structuration est au moins égale à 0,4 % du poids total dudit agent et mieux au moins égal à 0,9 %. En particulier, cette quantité est de 1,45 %.

De préférence, l'agent de structuration cationique comprend au moins un cation choisi parmi le calcium, le magnésium, l'aluminium, le sodium, le potassium, le lithium et leurs associations.

Ce ou ces cations sont normalement associés à un ou plusieurs contre-ions tels que ceux généralement associés à ce type de cations et en particulier des silicates.

Selon l'invention, l'agent de structuration est un solide hydraté ou non qui peut être d'origine naturelle ou synthétique. De préférence, on utilise des agents d'origine synthétique pour des raisons de reproductibilité dudit agent et de ses propriétés. Ces agents se présentent notamment sous forme de poudre.

Comme agent de structuration utilisable dans l'invention, on peut citer les silicates de sodium et de magnésium et en particulier le silicate de sodium, de lithium et de magnésium comme le produit vendu par la société Laporte sous le nom Laponite XLG, les silicates de magnésium et d'aluminium notamment hydratés comme le produit vendu par la société Vanderbilt Company sous le nom Veegum ultra, qui sont des argiles smectite synthétiques ou encore les silicates de calcium et notamment celui sous forme synthétique vendu par I société sous le nom de Micro-cel C.

De préférence, on utilise l'argile synthétique vendue sous le nom de Laponite XLG. En effet cette argile permet bien de conserver la dureté initiale de la composition notamment en stick, dans le temps, mais aussi d'augmenter de façon significative la brillance du film. L'argile synthétique vendue sous le nom de Veegum ultra ne fait que conserver la brillance initiale de la composition.

L'agent de structuration peut être introduit dans la composition avant que la composition ne durcisse, notamment sous forme de prégel à une concentration de 1 à 10 % dans une partie de la phase aqueuse de la composition. Il peut représenter de 0,1 à 10 % en matière active (M.A.) en poids par rapport au poids total de la composition et de préférence de 1 à 5 % en poids.

De façon avantageuse, l'oligomère et l'agent de structuration cationique sont présents dans un rapport pondéral oligomère/agent allant de 0,1 à 60 et mieux de 3 à 20.

L'invention s'applique non seulement aux produits de soin et/ou de traitement cosmétique des lèvres, des phanères et de la peau, y compris du cuir chevelu et l'intérieur des paupières, mais aussi aux produits de maquillage de la peau, des lèvres et des phanères ayant éventuellement des propriétés de soin et/ou de traitement. La composition peut être appliquée sur le visage, le cou, les mains, le corps, les lèvres, les ongles, les cils.

La composition selon l'invention peut se présenter sous la forme d'une composition dermatologique ou de soin de la peau, ou sous forme d'une composition de protection solaire ou de démaquillage. Elle se présente alors sous forme non colorée, contenant éventuellement des actifs cosmétiques ou dermatologiques. Elle peut alors être utilisée comme base de soin pour la peau ou les lèvres (baumes à lèvres protégeant les lèvres du froid et/ou du soleil et/ou du vent), comme déodorant ou encore comme produit de bronzage artificiel de la peau.

La composition de l'invention peut également se présenter sous la forme d'un produit coloré de maquillage de la peau, en particulier un fond de teint, un blush, un fard à joues ou à paupières, une eye liner, un mascara, un produit anti-cernes ou de maquillage des lèvres comme un rouge à lèvres de maquillage du corps, présentant des propriétés de soin ou de traitement. Dans ce cas, elle contient une ou plusieurs matières colorantes. Celles ci peuvent représenter de 0,1 à 40 % du poids total de la composition.

Bien entendu la composition de l'invention doit être cosmétiquement ou dermatologiquement acceptable, à savoir non toxique et susceptible d'être appliqué sur la peau, les lèvres et les phanères d'êtres humains.

La composition de l'invention comprend avantageusement une phase particulaire, généralement présente à raison de 0 à 35 % du poids total de la composition, de préférence de 5 à 25 %, et qui peut comprendre des pigments et/ou des nacres et/ou des charges habituellement utilisés dans les compositions cosmétiques.

La composition selon l'invention peut être fabriquée par les procédés connus, généralement utilisés dans le domaine cosmétique ou dermatologique.

L'invention a encore pour objet un procédé non thérapeutique et notamment cosmétique de soin, de traitement ou de maquillage de la peau, des lèvres ou des phanères des êtres humains, comprenant l'application sur la peau, les lèvres ou les phanères de la composition cosmétique telle que définie ci-dessus.

L'invention a encore pour objet un procédé non thérapeutique, pour diminuer, voire supprimer, le transfert d'un film d'une composition, déposé sur les lèvres et/ou sur la peau et/ou les phanères d'être humain, vers un support mis en contact avec le film, consistant à introduire dans la composition un agent gélifiant hydrophile de type polyester sulfoné et un agent de structuration cationique, ce polyester et cet agent de structuration cationique étant tels que définis précédemment.

L'invention a aussi pour objet l'utilisation de la composition ci-dessus pour l'obtention d'un film résistant à l'eau et/ou ayant des propriétés de non transfert et/ou de fraîcheur et/ou de confort et/ou ne laissant pas de traces et/ou de brillance.

L'invention a encore pour objet l'utilisation dans une composition cosmétique ou pour la fabrication d'une composition dermatologique, d'un agent gélifiant hydrophile de type polyester sulfoné et d'un agent de structuration cationique, ce polyester et cet agent de structuration cationique étant tels que définis précédemment.

L'invention est illustrée plus en détail dans les exemples suivants. Les pourcentages sont donnés en poids.

### Exemple 1 Préparation d'un oligomère copolyester téréphtalique

Dans un réacteur en acier inoxydable de 7,5 litres, muni d'un agitateur à ancre tournant à 80 tr/min relié à un couplemètre KYOWA, d'une double enveloppe pour la circulation d'un liquide caloporteur et d'une colonne à distiller régulée par une électrovanne on introduit :
- 11,47 moles de téréphtalate de diméthyle
- 2,53 moles de diméthylisophtalate-5 sulfonate de sodium
- 39,16 moles d'éthylène glycol
- 54 ppm en poids de titane, sous forme d'aminotriéthanolate de titane comme catalyseur et agent limiteur d'éthérification.

Le mélange est préchauffé à 180°C. Il est ensuite porté jusqu'à la température de 220°C en environ 130 min, pour distiller plus de 90% de la quantité théorique de méthanol. Le mélange réactionnel est ensuite amené à 230°C en 30 min. Lorsque la masse réactionnelle a atteint cette température, on introduit en 60 min, toujours à 230°C, une suspension dont la composition est la suivante :
- 0,5 mole d'acide isophtalique
- 2,36 moles d'acide téréphtalique
- 8 moles d'éthylène glycol.

La masse réactionnelle est alors amenée à la température de 250°C en 60 min. Pendant la période d'introduction du mélange et pendant la période de chauffage jusqu'à 250°C, on distille un mélange d'eau et d'éthylène glycol sans rétrogradation.

Le mélange réactionnel est ensuite transféré dans un autoclave préchauffé à 250°C puis mis sous pression réduite de 100 millibar en 22 min. Après 2 minutes dans ces conditions de température et de pression, la masse réactionnelle est coulée et refroidie.

Le copolyester obtenu présente les caractéristiques structurales décrites au tableau 1 ci-après, dans lequel:
∗ "% molaire des motifs diacides" correspond à la teneur, en %, de chaque diacide ou diester mise en oeuvre par rapport à l'ensemble des diacides ou diesters mis en oeuvre.
   "Tp" signifie : motif téréphtalique
   "lp" signifie : motif isophtalique
   "Slp" signifie : motif sulfoisophtalique.

Les caractéristiques de la partie "glycol" des copolyesters sont obtenues par méthanolyse des produits à 190°C pendant 16 h, suivie d'une analyse par la technique de chromatographie en phase vapeur et dosage par étalonnage interne.
- "% molaire des motifs diols" correspond à la teneur, en %, des motifs oxyéthylène "G", des motifs di(oxyéthylène) "2G", des motifs tri(oxyéthylène) "3G" et des motifs tétra(oxyéthylène) "4G", par rapport à l'ensemble des motifs diols.

* "%GT/S motifs" correspond au % molaire des motifs de formule (I)

   [-CO-A-CO-O-(CH₂-CH₂-O)ₙ-] (I')

   où A est 1,4 phénylène et n=1
   par rapport à l'ensemble des motifs de formule (I)
   où A est 1,4 phénylène, sulfo 1,3 phénylène et éventuellement 1,3 phénylène et n va de 1 à 4 "%GT/S motifs" se calcule par la formule suivante :
   %GT/S motifs = (% molaire de motifs Tp) x (% molaire de motifs G)/100.
* La masse molaire des polyesters (Mw) est déterminée par chromatographie par permation de gel (GPC) dans le DMAc/LiBr à 100%, les résultats sont données en équivalents polystyrène.

| % molaire des motifs diacides | |
|---|---|
| Tp | 82 |
| Ip | 3 |
| Slp | 15 |
| %GT/S motifs | 46,5 |

| % molaire des motifs diols | |
|---|---|
| G | 56,8 |
| 2G | 30,7 |
| 3G | 10 |
| 4G | 2,5 |
| Mw | 8 000 |

### Exemple 2 : de rouge à lèvres

On prépare la composition suivante:
- oligomère copolyester téréphtalique de l'exemple 1 20,0 g
- propylène glycol 1,96 g
- Laponite XLG 1,0 g
- pigments 4,0 g
- eau qsp 100

On forme tout d'abord un prégel à 5 % dans de l'eau de Laponite XLG, que l'on introduit dans l'eau contenant les pigments, sous agitation pendant 10min, puis on ajoute l'oligomère toujours sous agitation puis homogénéisation pendant 1h. La dureté finale du stick est obtenue au bout d'1 semaine et le film final de composition présente une brillance de 17. Ses propriétés de confort, de fraîcheur est de sans transfert sont remarquables.

La brillance peut être mesurée comme suit: dépôt d'un film de rouge de 100µm d'épaisseur sur une feuille de contraste, séchage 24 heures à température ambiante (25°C) puis mesure de la brillance avec un brillancemètre de type micro-Tri-Gloss, selon un angle de 60.

### Exemple 3 : rouge à lèvres

On prépare la composition suivante :
- oligomère copolyester téréphtalique de l'exemple 1 20,0 g
- propylène glycol 1,96 g
- Laponite XLG 3,0 g
- pigments 4,0 g
- eau qsp 100

Le mode opératoire est le même que celui de l'exemple 2.

La dureté du stick obtenue, au bout d'1 semaine est supérieure à celle de la composition de l'exemple 2 et le film final présente une brillance de 30. Ses propriétés de confort, de fraîcheur est de sans transfert sont remarquables.

## Revendications

1. Composition à application comprenant une phase aqueuse, un agent gélifiant hydrophile de type polyester sulfoné et un agent de structuration cationique choisi parmi les cations monovalents, multivalents, les polycations et leurs associations, ce polyester étant un oligomère copolyester téréphtalique hydrosoluble ou hydrodispersable de masse moléculaire, en poids, inférieure à 20 000 et comprenant essentiellement des motifs répétitifs dicarboxylates de formule (I):
[-CO-A-CO-O-(CH₂-CH₂O)ₙ-] (I)
dans laquelle
- A représente un groupement 1,4-phénylène, sulfo-1,3-phénylène et éventuellement 1,3-phénylène,
- n va de 1 à 4,
- au moins 35 % en mole desdits motifs de formule (I) étant des motifs pour lesquels A représente un groupement 1,4-phénylène et n est égal à 1,
- au moins 7 % en mole desdits motifs de formule (I) étant des motifs pour lesquels A représente un groupement sulfo-1,3-phénylène,
- et éventuellement jusqu'à 20 % en mole de motifs de formule (I) pour lesquels A représente un groupement 1,3-phénylène.

2. Composition selon la revendication 1, caractérisée par le fait que l'oligomère a une masse moléculaire en poids inférieure à 15 000.

3. Composition selon la revendication 1 ou 2, caractérisée par le fait que l'oligomère a une masse moléculaire en poids allant de 5 000 à 14 000, et mieux de 8 000 à 10 000.

4. Composition selon l'une quelconque des revendications précédentes, caractérisée par le fait que l'oligomère comprend au moins 40 % en mole desdits motifs de formule (I) pour lesquels A représente un groupement 1,4-phénylène et n est égal à 1.

5. Composition selon l'une quelconque des revendications précédentes, caractérisée par le fait que l'oligomère comprend de 40 à 90 % en mole desdits motifs de formule (I) pour lesquels A représente un groupement 1,4-phénylène et n est égal à 1.

6. Composition selon l'une quelconque des revendications précédentes, caractérisée par le fait que l'oligomère comprend au moins 10 % en mole des motifs de formule (I) pour lesquels A représente un groupement sulfo-1,3-phénylène.

7. Composition selon l'une quelconque des revendications précédentes, caractérisée par le fait que l'oligomère comprend de 10 % à 25 % en mole desdits motifs de formule (I) pour lesquels A représente un groupement sulfo-1,3-phénylène.

8. Composition selon l'une quelconque des revendications précédentes, caractérisée par le fait que l'oligomère comprend, en outre, de 0,5 % à 5 % en mole, de motifs de formule (I) pour lesquels A représente un groupement 1,3-phénylène.

9. Composition selon l'une quelconque des revendications précédentes, caractérisée par le fait que l'oligomère est présent en une teneur allant de 0,5 % à 40 % en poids, par rapport au poids total de la composition.

10. Composition selon l'une quelconque des revendications précédentes, caractérisée par le fait que l'oligomère est présent en une teneur allant de 5 % à 40 % en poids, par rapport au poids total de la composition, et mieux de 15 % à 25 % en poids.

11. Composition selon l'une quelconque des revendications précédentes, caractérisée par le fait que l'agent de structuration cationique comprend au moins 0,4 % en poids de cations ou polycations libres, par rapport au poids total dudit agent de structuration.

12. Composition selon l'une quelconque des revendications précédentes, caractérisée par le fait que l'agent de structuration cationique comprend au moins 0,9 % en poids de cations ou polycations libres, par rapport au poids total dudit agent de structuration.

13. Composition selon l'une quelconque des revendications précédentes, caractérisée par le fait que l'agent de structuration cationique comprend des cations choisis parmi le calcium, le magnésium, l'aluminium, le sodium, le potassium, le lithium et leurs associations.

14. Composition selon l'une quelconque des revendications précédentes, caractérisée par le fait que l'agent de structuration cationique comprend un contre-ion du type silicates.

15. Composition selon l'une quelconque des revendications précédentes, caractérisée par le fait que l'agent de structuration cationique est une argile d'origine synthétique.

16. Composition selon l'une quelconque des revendications précédentes, caractérisée par le fait que l'agent de structuration cationique est un silicate de magnésium, de lithium et de sodium.

17. Composition selon l'une quelconque des revendications précédentes, caractérisée par le fait que l'agent de structuration cationique représente de 0,1 à 10 % du poids total de la composition et mieux de 1 à 5%.

18. Composition selon l'une quelconque des revendications précédentes, caractérisée par le fait que l'oligomère et l'agent de structuration cationique sont présents dans un rapport pondéral oligomère/agent allant de 0,1 à 60 et mieux de 3 à 20.

19. Composition selon l'une quelconque des revendications précédentes, caractérisée par le fait que la composition est une composition de soin, de traitement ou de maquillage de la peau, des lèvres e/out des phanères.

20. Composition selon l'une quelconque des revendications précédentes, caractérisée par le fait que la composition contient au moins une matière colorante.

21. Composition selon l'une quelconque des revendications précédentes, caractérisée par le fait que la composition comprend au moins un additif choisi parmi les agents de protection solaire, les antioxydants, les conservateurs, les agents épaississants, les agents plastifiants, les tensioactifs, les charges, les cires, les huiles, les parfums, les actifs cosmétiques ou dermatologiques.

22. Composition selon l'une quelconque des revendications précédentes, caractérisée par le fait que la composition se présente sous la forme d'un produit de maquillage des lèvres, d'un fond de teint, d'un fard à paupières ou à joues, d'un mascara, d'un eye-liner, d'un produit anti-cernes, d'un produit de maquillage du corps, d'un produit de soin du visage, du cou, des mains, du corps, des ongles ou des lèvres, d'un produit déodorant, d'un produit de protection solaire.

23. Composition selon l'une des revendications précédentes, caractérisée en ce qu'elle se présente sous forme de stick.

24. Procédé non thérapeutique de soin, de traitement ou de maquillage de la peau, des lèvres ou des phanères des êtres humains, comprenant l'application sur la peau, les lèvres ou les phanères de la composition cosmétique selon l'une quelconque des revendications précédentes.

25. Procédé non thérapeutique, pour diminuer, voire supprimer, le transfert d'un film d'une composition cosmétique, déposé sur les lèvres et/ou sur la peau et/ou les phanères d'être humain, vers un support mis en contact avec le film, consistant à introduire dans la composition un agent gélifiant hydrophile de type polyester sulfoné et un agent de structuration cationique choisi parmi les cations monovalents ou multivalents, les polycations et leurs associations, ce polyester étant un oligomère copolyester téréphtalique hydrosoluble ou hydrodispersable de masse moléculaire en poids inférieure à 20 000 et comprenant essentiellement des motifs répétitifs dicarboxylates de formule (I):
[-CO-A-CO-O-(CH₂-CH₂O)ₙ-] (I')
dans laquelle
- A représente un groupement 1,4-phénylène, sulfo-1,3-phénylène et éventuellement 1,3-phénylène,
- n va de 1 à 4,
- au moins 35 % en mole desdits motifs de formule (I) étant des motifs pour lesquels A représente un groupement 1,4-phénylène et n est égal à 1,
- au moins 7 % en mole desdits motifs de formule (I) étant des motifs pour lesquels A représente un groupement sulfo-1,3-phénylène,
- et éventuellement jusqu'à 20 % en mole, de motifs de formule (I) pour lesquels A représente un groupement 1,3-phénylène.

26. Utilisation d'une composition selon l'une quelconque des revendications 1 à 23 pour l'obtention d'un film résistant à l'eau et/ou ayant des propriétés de non transfert et/ou de fraîcheur et/ou de confort et/ou ne laissant pas de traces et/ou de brillance.

27. Utilisation dans une composition cosmétique ou pour la fabrication d'une composition dermatologique, d'un agent gélifiant hydrophile de type polyester sulfoné et d'un agent de structuration cationique choisi parmi les cations monovalents, multivalents, les polycations et leurs associations, ce polyester étant un oligomère copolyester téréphtalique hydrosoluble ou hydrodispersable de masse moléculaire en poids inférieure à 20 000 et comprenant essentiellement des motifs répétitifs dicarboxylates de formule (I):
[-CO-A-CO-O-(CH₂-CH₂O)ₙ-] (I)
dans laquelle
- A représente un groupement 1,4-phénylène, sulfo-1,3-phénylène et éventuellement 1,3-phénylène,
- n va de 1 à 4,
- au moins 35 % en mole desdits motifs de formule (I) étant des motifs pour lesquels A représente un groupement 1,4-phénylène et n est égal à 1,
- au moins 7 % en mole desdits motifs de formule (I) étant des motifs pour lesquels A représente un groupement sulfo-1,3-phénylène,
- et éventuellement jusqu'à 20 % en mole, de motifs de formule (I) pour lesquels A représente un groupement 1,3-phénylène, pour obtenir un film confortable, brillant, sans transfert et/ ou de longue tenue et/ou ne laissant pas de traces et/ou ne migrant pas et/ou résistant à l'eau.
